(19) Europäisches Patentamt
European Patent Office
Office européen des brevets

(11) **EP 3 721 891 A1**

(12) **EUROPEAN PATENT APPLICATION**
published in accordance with Art. 153(4) EPC

(43) Date of publication:
**14.10.2020 Bulletin 2020/42**

(51) Int Cl.:
*A61K 36/43* (2006.01)  *A61K 36/62* (2006.01)
*A61K 36/288* (2006.01)  *A23L 33/105* (2016.01)

(21) Application number: **18885618.1**

(22) Date of filing: **06.12.2018**

(86) International application number:
**PCT/KR2018/015446**

(87) International publication number:
**WO 2019/112348 (13.06.2019 Gazette 2019/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.12.2017 KR 20170166860**

(71) Applicant: **Helixmith Co., Ltd**
**Gangseo-gu,**
**Seoul,**
**07794 (KR)**

(72) Inventors:
• **SON, Mi Won**
**Yongin-si**
**Gyeonggi-do 16822 (KR)**
• **LEE, Doo Suk**
**Anyang-si**
**Gyeonggi-do 14069 (KR)**
• **LEE, Won Woo**
**Seoul 05587 (KR)**
• **NAM, In Jeong**
**Seoul 07267 (KR)**
• **SON, Seung Hwan**
**Seoul 08329 (KR)**

(74) Representative: **J A Kemp LLP**
**14 South Square**
**Gray's Inn**
**London WC1R 5JJ (GB)**

(54) **HERBAL COMPOSITION FOR PREVENTING OR TREATING BENIGN PROSTATIC HYPERPLASIA DISEASE**

(57) The present invention relates to a composition for preventing or treating benign prostatic hyperplasia disease, the composition comprising, as active ingredients, extracts of *Semen Cuscutae*, *Semen nelumbinis*, and *Taraxaci Herba*. When using the present invention, it is possible to effectively prevent or treat benign prostatic hyperplasia without side effects.

Figure 3

## Description

Technical Field

This application claims priority to and the benefit of Korean Patent Application No. 10-2017-0166860 filed in the Korean Intellectual property Office on December 6, 2017, the entire contents of which are incorporated herein by reference.

The present disclosure relates to an herbal composition for preventing or treating benign prostatic hyperplasia disease. More specifically, the present disclosure relates to a composition for prevention or treatment of benign prostatic hyperplasia, which comprises a composite extract from *Semen Cuscutae, Semen nelumbinis,* and *Taraxaci Herba* and exhibits the function of suppressing an excessive increase in the size of the prostate gland and alleviating difficulty in discharge from the bladder.

Background Art

Benign prostatic hyperplasia (BPH) is one of prostate diseases, in which the prostate gland around the urethra increases in volume due to various factors, pressing on the urethra thereby making it difficult to pass urine out of the bladder. In recent years, benign prostatic hyperplasia is defined as a malady of lower urinary tract symptoms including frequent urination (eight or more times/day), nocturia, urgent urination (strong sudden need to urinate), urge incontinence (loss of bladder control), urinary hesitancy (a delay between trying to urinate and the flow actually beginning), intermittency (not continuous), involuntary interruption of voiding, etc. Testosterone and dihydrotestosterone (DHT) are considered to play a permissive role in prostate enlargement.

Medications for benign prostatic hyperplasia are largely divided into alpha blockers and androgen receptor inhibitors ($5\alpha$-reductase inhibitor). Alpha blockers act as drugs to reduce pressure and tension of the urethra of the prostate gland and include terazosin, doxazosin, tamsulosin, and alfuzosin, with side effects associated therewith reported to include dizziness, lack of energy, headaches, visual field defect, etc. Androgen receptor inhibitors, which are used as drugs for inhibiting $5\alpha$-reductase to prevent prostate enlargement, include finasteride and dutasteride and are reported to exhibit sexual function-related side effects.

*Semen Cuscutae* refers to seeds of *Cuscuta japonica, Cuscuta chienesis, Cuscuta australis,* or *Cuscuta pentagona,* which are all annual vine plants belonging to the family Convolvulaceae, and thus is also called cuscuta seed. *Semen Cuscutae* is known as an herbal medicine that acts to protect the liver and the kidney, improve eyesight, raise virility, and strengthen renal functions.

*Semen nelumbinis* refers to seeds of *Nelumbo nucifera Gaertner (Nelumbo nymphaea)* of the Nymphaeaceae family. The seeds are called renshi in Japanese. In China, the seed of the same plant is called lian zi and the dried young leaves and young roots within the mature seed are called lian zi xin. The seeds are used as a medicine mainly for treating symptoms associated with feeble spleen, such as diarrhea. The seeds are also applied to the treatment of the high heart beating symptom of insomnia.

*Taraxaci Herba* refers to *Taraxacum platycarpum H. Dahlsi* in the Asteraceae/Compositae family or the whole herb of plants in the same genus. In China, *Taraxacum monogolicum Hand.-Mazz,* or *Taraxacum sinicum Kitag* is used. Examples of the plants in the same genus include *Taraxacum coreanum Nakai, Taraxacum hallaisanensis Nakai, Taraxacum officinale Weber,* and *Taraxacum ohwianum Kitam. Taraxaci Herba* is used to decrease fever and applied to the treatment of swelling, mastitis, and laryngopharyngitis.

Detailed Description of the Invention

Technical Problem

Leading to the present disclosure, intensive and thorough research conducted by the present inventors into a natural substance-derived agent for prevention or treatment of benign prostatic hyperplasia, which is excellent in therapeutic effect, without side effects, resulted in the finding that a composition comprising a composite extract from *Semen Cuscutae, Semen nelumbinis,* and *Taraxaci Herba* has excellent efficacy in preventing or treating benign prostatic hyperplasia.

It is therefore a purpose of the present disclosure to provide a pharmaceutical composition for prevention or treatment of benign prostatic hyperplasia.

It is another purpose of the present disclosure to provide a food composition for alleviation of benign prostatic hyperplasia.

It is a further purpose of the present disclosure to provide a method for prevention or treatment of benign prostatic hyperplasia.

Other purposes and advantages of the present disclosure will become apparent from the following detailed description, claims, and drawings.

Technical Solution

According to an aspect thereof, the present disclosure provides a pharmaceutical composition for prevention or treatment of benign prostatic hyperplasia, the composition comprising (a) a composite extract from *Semen Cuscutae, Semen nelumbinis,* and *Taraxaci Herba* as an active ingredient; and (b) a pharmaceutically acceptable carrier.

In the present disclosure, *Semen Cuscutae* includes seeds of *Cuscuta japonica, Cuscuta chienesis, Cuscuta australis,* or *Cuscuta pentagona,* which are all annual vine plants belonging to the Convolvulaceae family.

In the present disclosure, *Semen nelumbinis* includes seeds of *Nelumbo nucifera Gaertner* (*Nelumbo nymphaea*) of the Nymphaeaceae family. The seeds are called renshi in Japanese. In China, the seed of the same plant is called lian zi and the dried young leaves and young roots within the mature seed are called lian zi xin.

In the present disclosure, *Taraxaci Herba* refers to *Taraxacum platycarpum H. Dahlsi* in the Asteraceae/Compositae family or the whole herb of plants in the same genus. Examples of the plants in the same genus include *Taraxacum monogolicum Hand.-Mazz, Taraxacum sinicum Kitag, Taraxacum coreanum Nakai, Taraxacum hallaisanensis Nakai, Taraxacum officinale Weber,* and *Taraxacum ohwianum Kitam.*

The composition of the present disclosure comprises a composite extract from *Semen Cuscutae, Semen nelumbinis,* and *Taraxaci Herba* as an active ingredient. The composite extract from *Semen Cuscutae, Semen nelumbinis,* and *Taraxaci Herba* of the present disclosure may be prepared (i) in a single extraction process in which extraction from a mixture of *Semen Cuscutae, Semen nelumbinis,* and *Taraxaci Herba* is made with an extraction solvent, or (ii) in a process in which respective extracts from *Semen Cuscutae, Semen nelumbinis,* and *Taraxaci Herba* are prepared and then mixed together.

In an embodiment of the present disclosure, the formulation weight ratio (w/w) of *Semen Cuscutae, Semen nelumbinis,* and *Taraxaci Herba* for the extract of the present disclosure is 1-4 : 1-4 : 1-4, based on weights of the ingredients prior to the extraction process. Particularly, for example, the formulation ratio of *Semen Cuscutae* : *Semen nelumbinis* : *Taraxaci Herba* may be 1-4 : 1 : 1, 1 : 1-4 : 1, or 1 : 1 : 1-4. More particularly, the formulation ratio of *Semen Cuscutae* : *Semen nelumbinis* : *Taraxaci Herba* may be 4 : 1 : 1, 1 : 4 : 1, or 1 : 1 : 4. In another embodiment, the formulation weight of *Semen Cuscutae* : *Semen nelumbinis* : *Taraxaci Herba* may be 1-2 : 1- 2 : 1-2, and particularly 1-2 : 1 : 1, 1 : 1-2 : 1, or 1 : 1 : 1-2, based on the weights of the ingredients prior to the extraction process. More particularly, the formulation ratio of *Semen Cuscutae* : *Semen nelumbinis* : *Taraxaci Herba* may be 2 : 1 : 1, 1 : 2 : 1, or 1 : 1 : 2.

As used herein, the term "formulation ratio" (w/w) refers to a weight ratio of individual ingredients prior art to an extraction process. For example, when the composite extract of the present disclosure is prepared in a single extraction process of subjecting a mixture of *Semen Cuscutae, Semen nelumbinis,* and *Taraxaci Herba* to extraction with an extraction solvent, the formulation ratio accounts for the weight ratio of the individual ingredients *Semen Cuscutae, Semen nelumbinis,* and *Taraxaci Herba* contained in the mixture. In addition, when the composite extract of the present invention is prepared in a process in which respective extracts from *Semen Cuscutae, Semen nelumbinis,* and *Taraxaci Herba* are prepared individually and then mixed, the formulation ratio accounts for the weight ratio based on "standard weights of individual ingredients" calculated according to the following arithmetic formula:

```
[Arithmetic Formula]
Standard weight of individual ingredient = weight
of individual ingredient used for individual extraction
x (weight of individual extract used for preparation of
final composite extract/weight of individual extract
prepared).
```

The extract used in the present disclosure can be obtained using a typical extraction solvent known in the art. The extraction solvent may be a polar or non-polar solvent. Examples of the polar solvent includes (a) water, (b) anhydrous or hydrous alcohol of 1-4 carbon atoms (e.g., methanol, ethanol, propanol, butanol, n-propanol, iso-propanol, and n-butanol), (c) acetic acid, and a mixture thereof. The non-polar solvent includes acetone, acetonitrile, ethyl acetate, methyl acetate, butyl acetate, fluoroalkane, hexane, ether, chloroform, dichloromethane, or a mixture thereof.

According to an embodiment of the present disclosure, the extract of the present disclosure is obtained using water or ethanol as an extraction solvent. For use as an extraction solvent in the present disclosure, ethanol includes not only absolute ethanol, but also an aqueous ethanol solution in a broad sense. The ethanol as an extraction solvent in the present disclosure may be 0 (exclusive) to 100 % ethanol in an embodiment, 0 (exclusive) to 95 % ethanol in another embodiment, 0 (exclusive) to 75 % ethanol in a further another embodiment, 0 (exclusive) to 50 % ethanol in a yet another embodiment, 5 to 30 % ethanol in a further yet another embodiment, 10 to 30 % ethanol in a still another embodiment, and 15 to 30 % ethanol in a still further another embodiment. More particularly, 25 % ethanol may be available.

The extract in the present disclosure may be obtained using a hot water extraction method, a cold-precipitation extraction method, a reflux condensation extraction method, an ultrasonic extraction method, or a typical method known in the art. In an embodiment, the extract of the present disclosure may be prepared using a cold precipitation extraction method. In a cold precipitation extraction method, extraction is conducted through cold precipitation for 1 to 72 hours, but without limitations thereto.

As used herein, the term "extract" is intended not only to be accepted as a crude extract in the art, but also to encompass a fraction obtained by additional fractionation of the crude extract in a broad sense. That is, the composite extract from *Semen Cuscutae, Semen nelumbinis,* and *Taraxaci Herba* includes a crude extract obtained using the above solvent, and a refined extract obtained by applying a filtration process to the crude extract. For example, fractions obtained through various purification methods, such as a fraction passing through an ultrafilter membrane with a predetermined molecular cut-off, fractions obtained by various chromatography methods (size, charge, hydrophobicity, affinity, etc.), etc. fall within the scope of the composite extract from *Semen Cuscutae, Semen nelumbinis,* and *Taraxaci Herba* of the present disclosure.

The composite extract from *Semen Cuscutae, Semen nelumbinis,* and *Taraxaci Herba* of the present disclosure may be prepared into powder by an additional procedure such as vacuum distillation, lyophilization, or spray drying.

The pharmaceutical composition of the present disclosure comprises a pharmaceutically effective amount of the composite extract from *Semen Cuscutae, Semen nelumbinis,* and *Taraxaci Herba.* As used herein, the term "pharmaceutically effective amount" refers to an amount effective in achieving the efficacy or activity of the composite extract from *Semen Cuscutae, Semen nelumbinis,* and *Taraxaci Herba.* No particular limitations are imparted to the amount if it can be used to achieve the purposes of the present disclosure.

As used herein, the term "prevention" refers to any action by which the onset of benign prostatic hyperplasia is inhibited or delayed through administration of a composition comprising the composite extract from *Semen Cuscutae, Semen nelumbinis,* and *Taraxaci Herba* of the present disclosure as an active ingredient.

As used herein, the term "treatment" refers to any action by which a symptom of benign prostatic hyperplasia takes a turn for the better or becomes modified favorably through administration of a composition comprising the composite extract from *Semen Cuscutae, Semen nelumbinis,* and *Taraxaci Herba* of the present disclosure as an active ingredient.

The pharmaceutical composition of the present disclosure comprises a pharmaceutically acceptable carrier. So long as it is typically used in the art, any pharmaceutically acceptable carrier may be available for the pharmaceutical composition of the present disclosure. Examples of the pharmaceutically acceptable carrier include lactose, dextrose, sucrose, sorbitol, mannitol, starch, acacia gum, calcium phosphate, alginate, gelatin, calcium silicate, microcrystalline cellulose, polyvinylpyrrolidone, water, syrup, and mineral oil, but are not limited thereto. In addition to the ingredient, the pharmaceutical composition of the present disclosure may further comprise a lubricant, a humectant, a sweetener, a flavorant, an emulsifier, a suspending agent, a preservative, and the like. With respect to suitable pharmaceutically acceptable carriers and agents, reference may be made to Remington's Pharmaceutical Sciences (19th ed., 1995).

The pharmaceutical composition of the present disclosure may be orally or parenterally (e.g., intravenously, intraperitoneally, intramuscularly, subcutaneously, or topically) administered and preferably orally.

An appropriate dosage of the pharmaceutical composition of the present disclosure may vary depending on various factors including formulation methods, administration types, age, body weight, sex, and morbidity of patients, diets, administration time, administration route, excretion rate, and response sensitivity. A general dose of the pharmaceutical composition of the present disclosure falls within the range of 0.001-1000 mg/kg for adults. In addition, the dose for the human body may be calculated on the basis of animal tests (Shin et al., J Korean Oriental Medicine 31(3):1-7, 2010).

The pharmaceutical composition of the present disclosure may be formulated as general formulations using the pharmaceutically acceptable carriers and/or excipients according to methods easily practiced by a person skilled in the art to which the present disclosure belongs, to be prepared as a unit dosage form or to be prepared by introducing the composition into a multi-dosage container. In this regard, the general formulation may be in the form of a solution in oil or an aqueous medium, a suspension, a syrup, or an emulsion or in the form of an extract, powders, granules, a tablet, or a capsule and may further comprise a dispersant or a stabilizer.

According to another aspect thereof, the present disclosure provides a food composition comprising a composite extract from *Semen Cuscutae, Semen nelumbinis,* and *Taraxaci Herba* as an active ingredient for alleviation of benign prostatic hyperplasia.

As used herein, the term "alleviation" refers to any action by which a symptom of benign prostatic hyperplasia is reduced to at least some degree through administration of a composition comprising the composite extract from *Semen*

*Cuscutae, Semen nelumbinis,* and *Taraxaci Herba* of the present disclosure as an active ingredient.

When prepared into a food composition, the composition of the present disclosure may comprise an ingredient typically used in food preparation, such as a protein, a carbohydrate, a lipid, a nutrient, a seasoning, and a flavorant. Examples of the carbohydrate include monosaccharides, e.g., glucose, fructose, and the like; disaccharides, e.g., maltose, sucrose, and the like; oligosaccharides; and polysaccharides, e.g., dextrin, cyclodextrin, and the like; and sugar alcohols such as xylitol, sorbitol, erythritol, etc. The flavorant may be a natural flavorant or a synthetic flavorant. By way of example, when prepared into a drink, the food composition of the present disclosure may further comprise citric acid, liquid fructose, sugar, glucose, acetic acid, malic acid, fruit juice, etc. in addition to the active ingredient.

For the food composition of the present disclosure, the composite extract from *Semen Cuscutae, Semen nelumbinis,* and *Taraxaci Herba,* which is contained in the pharmaceutical composition according to an aspect of the present disclosure, may be employed per se. The description of the overlapping content in the relationship with the pharmaceutical composition is omitted to avoid excessive complexity of the present disclosure.

According to a further aspect thereof, the present disclosure provides a method for treatment of benign prostatic hyperplasia, the method comprising administering to a subject in need thereof a pharmaceutically effective amount of a pharmaceutical composition comprising a composite extract from *Semen Cuscutae, Semen nelumbinis,* and *Taraxaci Herba* as an active ingredient.

As used herein, the term "subject" refers to an animal that is suspected or diagnosed to undergo the onset of benign prostatic hyperplasia.

The subject to which the treatment method of the present disclosure is applied is a mammal as exemplified by a human, a mouse, a rat, a guinea pig, a dog, a cat, a horse, a cow, a pig, a monkey, a chimpanzee, a baboon, and a rhesus. Most particularly, the subject of the present disclosure is a human.

In the method for treatment of benign prostatic hyperplasia according to the present disclosure, the treatment of benign prostatic hyperplasia is conducted by administering the pharmaceutical composition according to an aspect of the present disclosure to a subject. Hence, the description of the pharmaceutical composition is incorporated in the description of the treatment method of the present disclosure by reference, and the overlapping content is omitted to avoid excessive complexity of the present disclosure.

Brief Description of the Drawings

Features and advantages of the present disclosure are summarized as follows:

(a) The present disclosure provides a pharmaceutical composition for prevention or treatment of benign prostatic hyperplasia.
(b) The present disclosure provides a food composition for alleviation of benign prostatic hyperplasia.
(c) The present disclosure provides a method for prevention or treatment of benign prostatic hyperplasia.
(d) The pharmaceutical composition of the present disclosure can be used in effectively preventing or treating benign prostatic hyperplasia, without side effects.

Brief Description of the Drawings

FIG. 1 is a plot showing inhibitory effects of composite herbal extracts on testosterone-induced prostate epithelial cells (LNCaP).
FIG. 2 is a graph showing inhibitory effects of composite herbal extracts prepared with solutions of various ethanol concentrations on testosterone-induced prostate epithelial cells (LNCaP).
FIG. 3 is a graph showing inhibitory effects of the composite herbal extract and the single herbal extracts on testosterone-induced prostate epithelial cells (LNCaP).
FIG. 4 is a plot showing testosterone-induced benign prostatic hyperplasia rat models changing in body weight with treatment with the composite herbal extract or the single herbal extracts.
FIG. 5 is a graph showing inhibitory effects of the composite herbal extract and the single herbal extracts on prostate enlargement in benign prostatic hyperplasia-induced rat models.
FIG. 6 shows sizes of acinus in the prostate tissues excised from testosterone-induced benign prostatic hyperplasia rat models after treatment with the composite herbal extract and the single herbal extracts.

Mode for Carrying Out the Invention

A better understanding of the present disclosure may be obtained through the following examples which are set forth to illustrate, but are not to be construed as limiting the present disclosure.

**EXAMPLES**

**PREPARAITON EXAMPLE 1: Preparation of composite herbal extracts by various formulation ratios**

*Semen Cuscutae, Semen nelumbinis,* and *Taraxaci Herba* were purchased from Humanherb Co. Ltd., which is an herb distributor in Korea, and washed and dried before use in experiments. The medicinal herbs *Semen Cuscutae, Semen nelumbinis,* and *Taraxaci Herba* were mixed at the weight ratios (w/w) indicated in Table 1, below, to form mixtures each weighing 240 g in total. Each of the mixtures was added with 10 volumes of a 25% ethanol solution and then well stirred at room temperature for 72 hours to conduct extraction. The extract thus formed was filtered, concentrated at 50-65°C in a vacuum, and lyophilized to afford a total of seven composite herbal extract powders. Their yields are given in Table 1, below.

TABLE 1

| Preparation Ex. # | *Semen Cuscutae* | *Semen nelumbinis* | *Taraxaci Herba* | Yield (%) |
|---|---|---|---|---|
| 1-1 | 1 | 1 | 1 | 11.35 |
| 1-2 | 2 | 1 | 1 | 11.22 |
| 1-3 | 4 | 1 | 1 | 10.17 |
| 1-4 | 1 | 2 | 1 | 12.08 |
| 1-5 | 1 | 4 | 1 | 12.39 |
| 1-6 | 1 | 1 | 2 | 11.71 |
| 1-7 | 1 | 1 | 4 | 10.85 |

**PREPARAITON EXAMPLE 2: Preparation of composite herbal extracts by various extraction times**

*Semen Cuscutae, Semen nelumbinis,* and *Taraxaci Herba* were purchased from Humanherb Co. Ltd., which is an herb distributor in Korea, and washed and dried before use in experiments. The medicinal herbs *Semen Cuscutae, Semen nelumbinis,* and *Taraxaci Herba* were mixed at the weight ratio (w/w) of Preparation Example 1-6 to form a mixture weighing 240 g. The mixture was added with 10 volumes of a 25% ethanol solution and then well stirred at room temperature for 0.5, 3, 8, 24, or 72 hours to conduct extraction. The extract thus formed was filtered, concentrated at 50-65°C in a vacuum, and lyophilized to afford a total of five composite herbal extract powders. Their yields are given in Table 2, below.

TABLE 2

| Preparation Ex. # | Extraction time (hr) | Yield (%) | Note |
|---|---|---|---|
| 2-1 | 0.5 | 9.04 | |
| 2-2 | 3 | 10.87 | |
| 2-3 | 8 | 10.01 | |
| 2-4 | 24 | 11.17 | |
| 2-5 | 72 | 11.71 | the same as in Preparation Example 1-6 |

**PREPARAITON EXAMPLE 3: Preparation of composite herbal extracts by various ethanol concentrations**

*Semen Cuscutae, Semen nelumbinis,* and *Taraxaci Herba* were purchased from Humanherb Co. Ltd., which is an herb distributor in Korea, and washed and dried before use in experiments. The medicinal herbs *Semen Cuscutae, Semen nelumbinis,* and *Taraxaci Herba* were mixed at the weight ratio (w/w) of Preparation Example 1-6 to form a mixture weighing 240 g. The mixture was added with 10 volumes of a 0, 10, 25, 50, 75, or 95% ethanol solution and then well stirred at room temperature for 72 hours to conduct extraction. The extract thus formed was filtered, concentrated at 50-65°C in a vacuum, and lyophilized to afford a total of six composite herbal extract powders. Their yields are given in Table 3, below.

TABLE 3

| Preparation Ex. # | Conc. of Ethanol solution (%) | Yield (%) | Note |
|---|---|---|---|
| 3-1 | 0 | 10.32 | |
| 3-2 | 10 | 10.63 | |
| 3-3 | 25 | 11.71 | the same as in Preparation Example 1-6 or 2-5 |
| 3-4 | 50 | 12.28 | |
| 3-5 | 75 | 8.81 | |
| 3-6 | 95 | 5.16 | |

**PREPARAITON EXAMPLE 4: Preparation of Composite Herbal Extract through Hot Water Extraction**

*Semen Cuscutae, Semen nelumbinis,* and *Taraxaci Herba* were purchased from Humanherb Co. Ltd., which is an herb distributor in Korea, and washed and dried before use in experiments. The medicinal herbs *Semen Cuscutae, Semen nelumbinis,* and *Taraxaci Herba* were mixed in the respective amounts of 30 g, 30 g, and 60 g at the weight ratio (w/w) of Preparation Example 1-6 to form a mixture. The mixture was added with 10 volumes of distilled water and then well stirred at 95-100°C for 3 hours to conduct extraction under reflux. The extract thus formed was filtered, concentrated at 50-65°C in a vacuum, and lyophilized to afford a composite herbal extract powder. The yield was about 11.90 %.

**COMPARATIVE EXAMPLE 1: Preparation of Extract from Single Herb**

*Semen Cuscutae, Semen nelumbinis,* and *Taraxaci Herba* were purchased, washed, and dried for use in experiments. To 180 g of each of the herbs, 10 volumes of 25% ethanol solution were added, followed by stirring at room temperature for 72 hours to conduct extraction. The extract thus formed was filtered, concentrated at 50-65°C in a vacuum, and lyophilized to afford a total of three single herbal extract powders. Their yields are given Table 4, below.

TABLE 4

| Comparative Ex. # | Herb | Yield (%) |
|---|---|---|
| 1-1 | *Semen Cuscutae* | 5.49 |
| 1-2 | *Semen nelumbinis* | 16.35 |
| 1-3 | *Taraxaci Herba* | 15.00 |

**EXPERIMENTAL EXAMPLE 1: Test of Inhibitory Activity of Composite Herbal Extract against Testosterone-Induced Proliferation of Prostate Epithelial Cell Line**

(Test method)

LNCaP cells, which are the prostate epithelial cells derived from human prostate cancer, were seeded at a density of $1 \times 10^5$ cells/well in 24-well plates containing an RPMI medium (Gibco U. S. A.) and stabilized overnight. Then, the cells were cultured for 24 hours in a serum-free medium. After removal of the medium, the cells were incubated for 72 hours with 2 $\mu$M of testosterone (Tes) alone or in combination with 50, 100, or 200 $\mu$g/ml of the composite herbal extract of Preparation Example 1-6 or 4. Thereafter, growth of the cells was evaluated by MTT assay. Cell proliferation data were expressed as percentages of the cell counts relative to that of the Tes-treated group.

(Results)

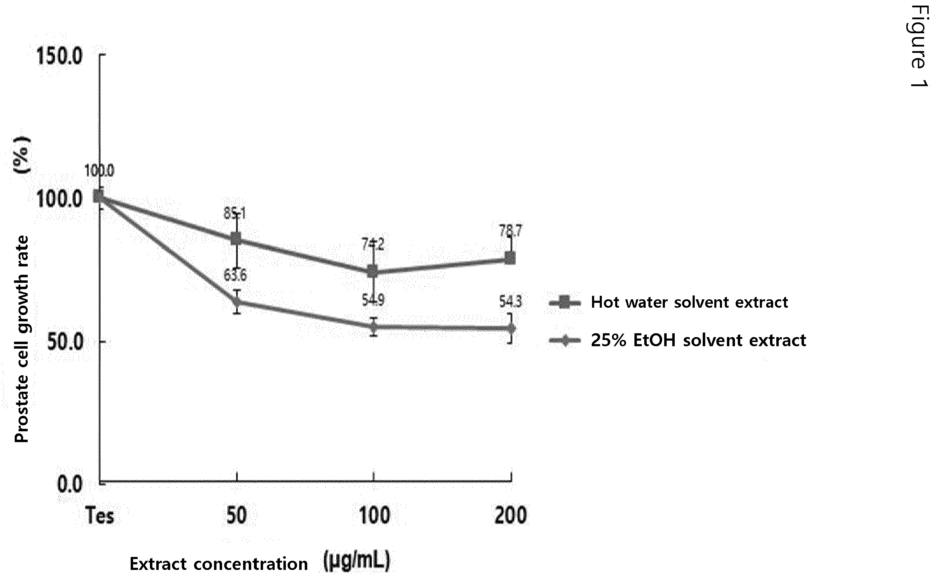

When comparison was made of effects between the composite herbal extracts prepared with hot water (Preparation Example 4) and 25% ethanol solution (Preparation Example 1-6), the two extracts were both observed to inhibit prostate cell growth in a dose dependent manner (FIG. 1).

**EXPERIMENTAL EXAMPL 2: Test of Inhibitory Activity of Composite Herbal Extract Prepared According to Various Ethanol Concentrations against Testosterone-Induced Proliferation of Prostate Epithelial Cell Line**

(Test Method)

LNCaP cells, which are the prostate epithelial cells derived from human prostate cancer, were seeded at a density of $1\times10^5$ cells/well in 24-well plates containing an RPMI medium (Gibco, U. S. A.) and stabilized overnight. Then, the cells were cultured for 24 hours in a serum-free medium. After removal of the medium, the cells were incubated for 72 hours with 2 $\mu$M of testosterone (Tes) alone or in combination with 200 $\mu$g/ml of composite herbal extract of Preparation Example 1-6, or each of the three kinds of single herbal extract of Comparative Example 1. Thereafter, growth of the cells was evaluated by MTT assay. Cell proliferation data were expressed as percentages of the cell counts relative to that of the Tes-treated group.

(Results)

Effects of the composite herbal extracts prepared according to various ethanol concentrations on testosterone-induced proliferation of prostate epithelial cells are given in Table 5, below.

TABLE 5

| Substance | Cell growth (% of control) | % inhibition of cell growth |
|---|---|---|
| NC (not treated) | - | - |
| Tes (Testosterone) | 100 | - |
| Testosterone + Preparation Ex. 3-1 | 45.91 | 54.09 |
| Testosterone + Preparation Ex. 3-2 | 41.16 | 58.84 |
| Testosterone + Preparation Ex. 3-3 | 34.59 | 65.41 |
| Testosterone + Preparation Ex. 3-4 | 58.43 | 41.57 |
| Testosterone + Preparation Ex. 3-5 | 74.00 | 26.00 |
| Testosterone + Preparation Ex. 3-6 | 83.54 | 16.46 |

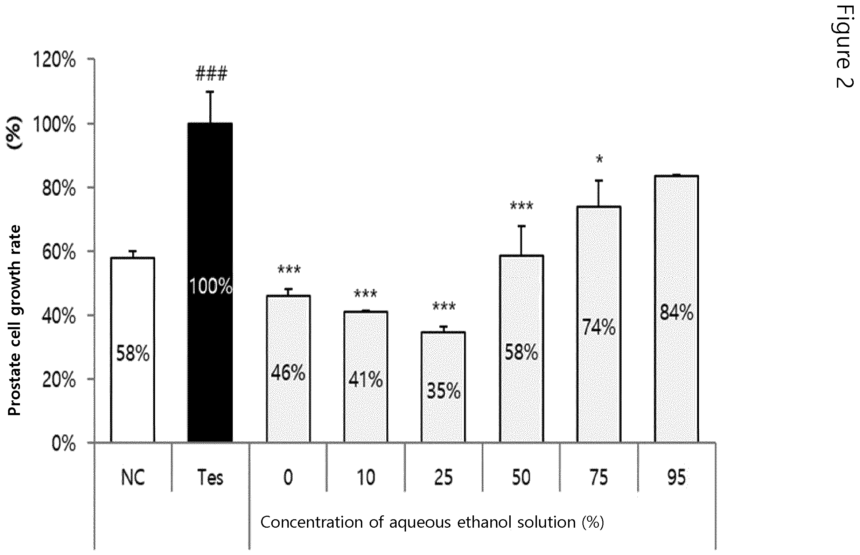

Comparison of effects of the composite herbal extracts according to various ethanol concentrations showed that all the cell groups treated with of the extracts prepared in Preparations Examples 3-1 to 3-6 were lower in cell growth rate than the Tes-treated group, demonstrating that the composite herbal extracts prepared at various ethanol concentrations have excellent inhibitory activity against the growth of prostate cells (FIG. 2).

**EXPERIMENTAL EXAMPLE 3: Comparison of Inhibitory Activity against Testosterone-Induced Proliferation of Prostate Epithelial Cell Line between Composite Herbal Extract and Individual Herbal Extract**

(Experimental Method)

LNCaP cells, which are the prostate epithelial cells derived from human prostate cancer, were seeded at a density of $1\times10^5$ cells/well in 24-well plates containing an RPMI medium (Gibco, U. S. A.) and stabilized overnight. Then, the cells were cultured for 24 hours in a serum-free medium. After removal of the medium, the cells were incubated for 72 hours with 2 $\mu$M of testosterone (Tes) alone or in combination with 200 $\mu$g/ml of each of the six composite herbal extracts of Preparation Example 3 or each of the three single herbal extracts. Thereafter, growth of the cells was evaluated by MTT assay. Cell proliferation data were expressed as percentages of the cell counts relative to that of the Tes-treated group.

(Results)

Effects of the composite herbal extracts and the single herbal extracts on testosterone-induced proliferation of prostate epithelial cells are given in Table 6, below.

TABLE 6

| | 200 $\mu$g/ml | |
| --- | --- | --- |
| | Cell growth rate (%) | % Inhibition of cell growth |
| NC | - | - |
| Testosterone | 100.0 | - |
| Preparation Ex.1-6 | 44.9 | 55.1 |
| Comparative Ex.1-1 (*Semen Cuscutae*) | 66.7 | 33.3 |
| Comparative Ex. 1-2 (*Semen nelumbinis*) | 78.0 | 22.0 |
| Comparative Ex. 1-3 *(Taraxaci Herba)* | 88.1 | 11.9 |

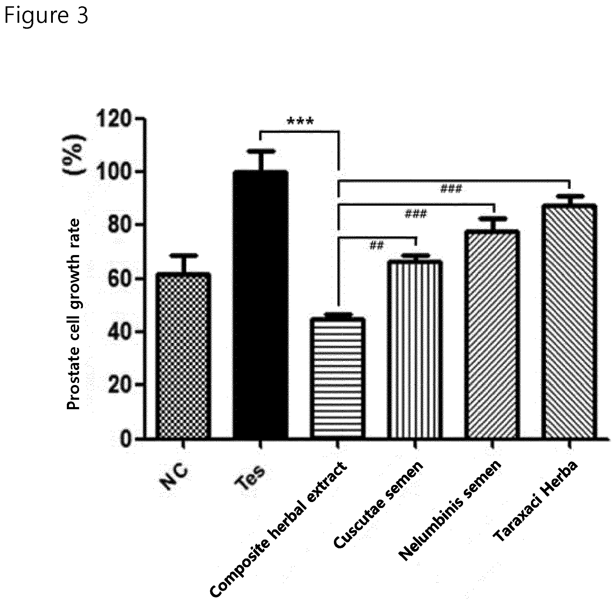

When treated with testosterone, the prostate epithelial cells were measured to grow by about 62.5%. Treatment with the composite herbal extract of Preparation Example 1-6 together with testosterone inhibited the cell growth by about 55.1%. In contrast, the single herbal extracts of Comparative Example 1-1 (*Semen Cuscutae*), Comparative Example 1-2 (*Semen nelumbinis*), and Comparative Example 1-3 (*Taraxaci Herba*) inhibited the growth of the cells by about 33.3%, 22.0%, and 11.9%, respectively. Thus, the composite herbal extract was observed to be far superior to the single herbal extracts in terms of growth inhibition at the same ethanol concentration (FIG. 3).

**EXPERIMENTAL EXAMPLE 4: Comparison of Inhibitory Activity against Prostate Enlargement between Single Herbal Extracts and Composite Herbal Extract in Testosterone-Induced Benign Prostatic Hyperplasia Rat Model**

(Test Method)

After being acclimated for one week or longer, male SD rats 9 weeks old (weighing 350 g or less) were divided on the basis of weight into (1) a normal group, (2) a benign prostatic hyperplasia-induced and distilled water-administered group (negative control), (3) benign prostatic hyperplasia-induced and composite herbal extract-administered group (Preparation Example 1-6), (4) benign prostatic hyperplasia-induced and single *Semen Cuscutae* herbal extract-administered group (Comparative Example 1-1), (5) benign prostatic hyperplasia-induced and single *Semen nelumbinis* herbal extract-administered group (Comparative Example 1-2), (6) benign prostatic hyperplasia-induced and single *Taraxaci Herba* herbal extract-administered group (Comparative Example 1-3), and (7) benign prostatic hyperplasia-induced and finasteride-administered group (positive control). In order to induce benign prostatic hyperplasia, testosterone (dissolved in cotton seed oil) was subcutaneously injected at a dose of 3 mg/kg once/three days for a total of 10 times. For the control, the same amount of cotton seed oil was subcutaneously injected. The composite herbal extract of Preparation Example 1-6 and the single herbal extracts of Comparative Example 1 were each orally administered at a dose of 300 mg/kg once a day for 30 days. Finasteride (Sigma, dissolved in 0.2% Tween 80) was orally administered at a dose of 10 mg/kg once/day for 30 days. For the negative control, oral administration of distilled water alone was carried out. From the starting day of the test, the rats were weighed five times in total at regular intervals of one week. On the next day after the final administration and treatment, the rats were sacrificed with carbon dioxide gas. The prostate glands were excised and weighed. % Inhibition of prostate enlargement was expressed according to the following mathematical formula on the basis of the weight measurements.

```
     Mathematical Formula
     % Inhibition of prostate enlargement = 100 -
{(prostate   weight   of   composite   herbal   extract
group/single herbal extract or positive control group -
prostate  weight  of  control  group)  *  100  /  (prostate
weight of negative control - prostate weight of normal
group)}
```

In addition, the excised prostate tissue was fixed in 10% neutral buffered formalin and subjected to H&E (hematoxylin and eosin) staining. Slides of the stained tissues were observed and photographed with an optical microscope. From the images, sizes of the acini in the prostate glands were measured. The reduction in the size of acinus of each experimental group is expressed as a percentage relative to the size of acinus of the normal group.

(Results)

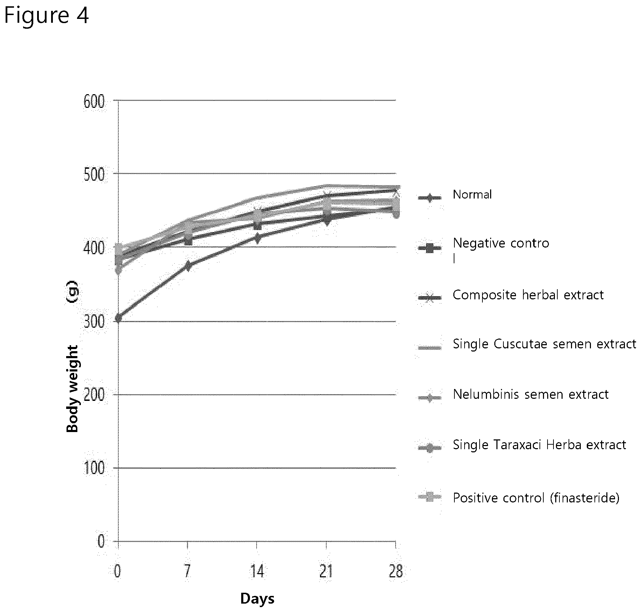

Benign prostatic hyperplasia was induced. The normal group steadily increased in body weight whereas the increase of body weight was suppressed in the groups treated with testosterone. However, significant differences in body weight were not observed among the negative control, the composite herbal extract group (Preparation Example 1-6), the single herbal extract groups (Comparative Examples 1-1, 1-2, and 1-3), and the positive group (FIG. 4). Effects of the composite herbal extract (Preparation Example 1-6), the single herbal extracts (Comparative Examples 1-1, 1-2, and 1-3), and the positive control on the testosterone-induced prostate enlargement are given in Table 7, below.

TABLE 7

| Experimental Group | Prostate weight/body weight (g/kg) | % Inhibition of prostate enlargement |
|---|---|---|
| Normal | $0.08 \pm 0.01$ | - |
| Negative control | $2.26 \pm 0.21$ | - |
| Composite herbal extract | $1.49 \pm 0.26$ | 35 |
| Single *Semen Cuscutae* extract | $1.82 \pm 0.09$ | 20 |
| Single *Semen nelumbinis* extract | $1.79 \pm 0.20$ | 21 |
| Single *Taraxaci Herba* extract | $2.14 \pm 0.17$ | 5 |
| Positive control (Finasteride) | $1.31 \pm 0.10$ | 43 |

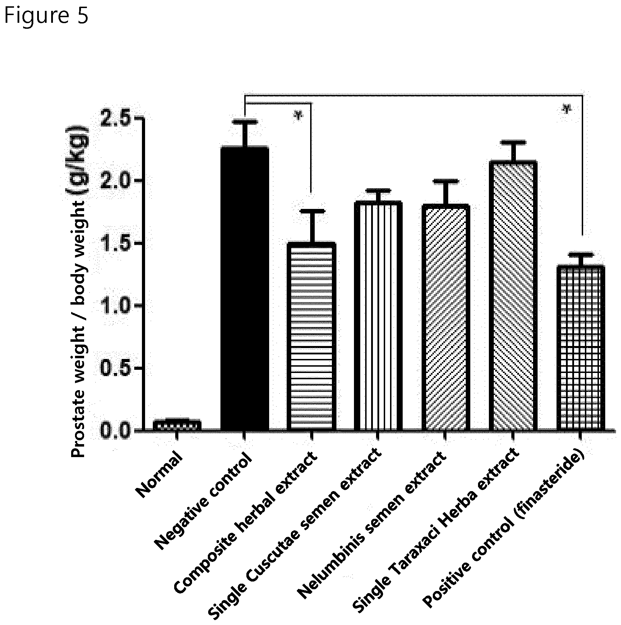

The rats in which benign prostatic hyperplasia was induced by testosterone increased in prostate weight by about 30 fold compared to the normal group. However, the prostate weight in the composite herbal extract group was decreased by 35 % compared to that in the testosterone-induced group (negative control), with a statistically significant difference. In contrast, the single herbal extract groups (Comparative Examples 1-1(*Semen Cuscutae),* 1-2 (*Semen nelumbinis*), and 1-3 (*Taraxaci Herba*)) exhibited inhibitory effects on prostate enlargement by about 20%, 21%, and 5%, respectively, compared to the testosterone-induced group (negative control), but without a statistically significant difference (FIG. 5). The data indicate that the composite herbal extract exerts a far higher inhibitory activity against testosterone-induced prostate enlargement, compared to the single herbal extracts.

Effects of the composite herbal extract (Preparation Example 1-6), the single herbal extracts (Comparative Examples 1-1, 1-2, and 1-3), and the positive control on the size of acinus are summarized in Table 8, below.

TABLE 8

| Experimental Group | Size of acinus (%) | Reductive effect on size of acinus (%) |
|---|---|---|
| Normal | $100 \pm 30$ | - |
| Negative control | $481 \pm 52$ | - |
| Composite herbal extract | $270 \pm 48$ | 44 |
| Single *Semen Cuscutae* extract | $375 \pm 25$ | 22 |
| Single *Semen nelumbinis* extract | $334 \pm 18$ | 30 |
| Single *Taraxaci Herba* extract | $482 \pm 65$ | 0 |
| Positive control (Finasteride) | $279 \pm 10$ | 42 |

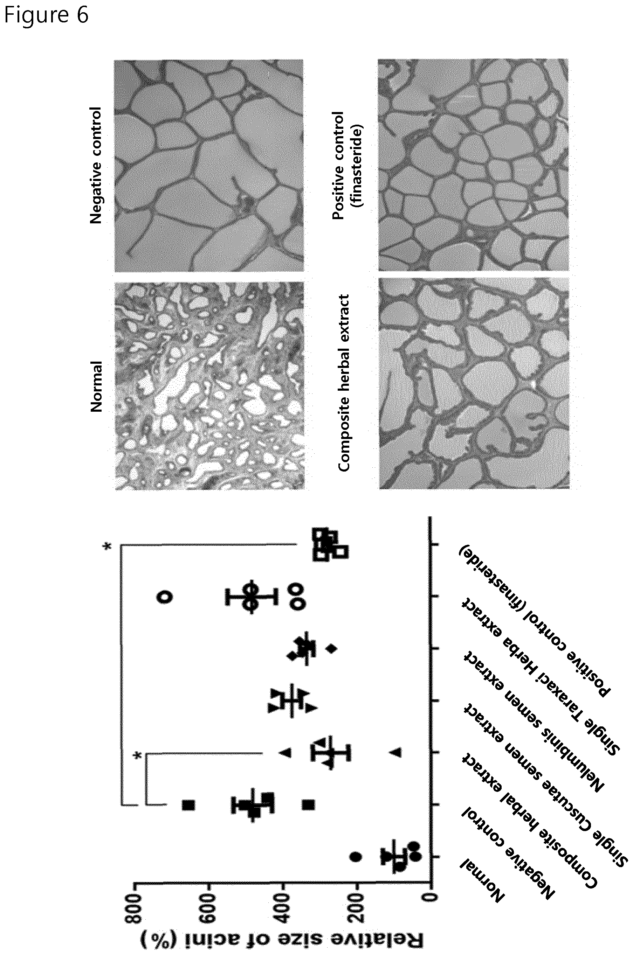

The negative control increased in the size of acinus by 4.8 fold, compared to the normal group while the composite herbal extract exhibited the effect of reducing the size of acinus by 44 %, with statistical significance. For the single herbal extracts (Comparative Examples 1-1, 1-2, and 1-3), the reduction rates were 22, 30, and 0%, respectively, which are far short from the effect of the composite herbal extract. Taken together, the data demonstrate that the composite herbal extract has a far higher inhibitory activity against testosterone-induced prostate enlargement, compared to the single herbal extracts (FIG. 6).

**Claims**

1. A pharmaceutical composition for prevention or treatment of benign prostatic hyperplasia, the composition comprising:

   (a) a composite extract from *Semen Cuscutae, Semen nelumbinis,* and *Taraxaci Herba* as an active ingredient; and
   (b) a pharmaceutically acceptable carrier.

2. The pharmaceutical composition of claim 1, wherein the *Semen Cuscutae,* the *Semen nelumbinis,* and the *Taraxaci Herba* are mixed at a formulation weight ratio (w/w) of 1-4 : 1-4 : 1-4.

3. The pharmaceutical composition of claim 1, wherein the extract is a water or ethanol extract.

4. The pharmaceutical composition of claim 3, wherein the ethanol is 0 (exclusive) - 50 % ethanol.

5. The pharmaceutical composition of claim 3, wherein the ethanol is 25 % ethanol.

6. A food composition comprising a composite extract from *Semen Cuscutae, Semen nelumbinis,* and *Taraxaci Herba* as an active ingredient for alleviation of benign prostatic hyperplasia.

7. The food composition of claim 6, wherein the *Semen Cuscutae,* the *Semen nelumbinis,* and the *Taraxaci Herba* are mixed at a formulation weight ratio (w/w) of 1-4 : 1-4 : 1-4.

Figure 1

Figure 2

Figure 3

# Figure 4

Figure 5

# Figure 6

## INTERNATIONAL SEARCH REPORT

| International application No. |
| --- |
| **PCT/KR2018/015446** |

**A.    CLASSIFICATION OF SUBJECT MATTER**

*A61K 36/43(2006.01)i, A61K 36/62(2006.01)i, A61K 36/288(2006.01)i, A23L 33/105(2016.01)i*

According to International Patent Classification (IPC) or to both national classification and IPC

**B.    FIELDS SEARCHED**

Minimum documentation searched (classification system followed by classification symbols)
A61K 36/43; A23L 1/30; A61K 35/78; A61K 36/17; A61K 36/254; A61K 36/288; A61K 36/489; A61K 36/734; A61K 7/00; A61K 7/06; A61K 36/62; A23L 33/105

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: benign prostatic hyperplasia, taraxaci herba, cuscutae semen, nelumbinis semen, extract, ethanol, taraxacum platycarpum dahlst., PROSTATE, benign prostatic hyperplasia

**C.    DOCUMENTS CONSIDERED TO BE RELEVANT**

| Category* | Citation of document, with indication, where appropriate, of the relevant passages | Relevant to claim No. |
| --- | --- | --- |
| Y | KR 10-0832404 B1 (NEWGEX INC.) 26 May 2008<br>See abstract; paragraphs [0008], [0021], [0024]; and claims 7, 10. | 1-7 |
| Y | KR 10-2013-0081131 A (KNU-INDUSTRY COOPERATION FOUNDATION)<br>16 July 2013<br>See abstract; paragraphs [0036]-[0047]; and claims 6, 9, 10. | 1-7 |
| A | KR 10-2013-0088333 A (INDUSTRY ACADEMIC COOPERATION FOUNDATION, HALLYM UNIVERSITY) 08 August 2013<br>See abstract; and claims 1-3. | 1-7 |
| A | JP 2000-095648 A (TSUMURA & CO.) 04 April 2000<br>See abstract; paragraph [0004]; and claims 1, 2. | 1-7 |
| A | JP 2002-087976 A (MARUZEN PHARMACEUT CO., LTD.) 27 March 2002<br>See abstract; and claims 1, 5. | 1-7 |

| ☐ | Further documents are listed in the continuation of Box C. | ☒ | See patent family annex. |
| --- | --- | --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 03 APRIL 2019 (03.04.2019) | **03 APRIL 2019 (03.04.2019)** |

| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |
| --- | --- |

Form PCT/ISA/210 (second sheet) (January 2015)

INTERNATIONAL SEARCH REPORT
Information on patent family members

International application No.

**PCT/KR2018/015446**

| Patent document cited in search report | Publication date | Patent family member | Publication date |
|---|---|---|---|
| KR 10-0832404 B1 | 26/05/2008 | KR 10-2007-0118921 A | 18/12/2007 |
| KR 10-2013-0081131 A | 16/07/2013 | KR 10-1381831 B1 | 14/04/2014 |
| KR 10-2013-0088333 A | 08/08/2013 | KR 10-1407659 B1 | 13/06/2014 |
| JP 2000-095648 A | 04/04/2000 | JP 4033981 B2 | 16/01/2008 |
| JP 2002-087976 A | 27/03/2002 | JP 4672124 B2 | 20/04/2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020170166860

**Non-patent literature cited in the description**

- Remington's Pharmaceutical Sciences. 1995

- **SHIN et al.** *J Korean Oriental Medicine,* 2010, vol. 31 (3), 1-7